# EUROPÄISCHE PATENTSCHRIFT

(11) **EP 1 863 549 B1**
(45) Veröffentlichungstag und Bekanntmachung des Hinweises auf die Patenterteilung: **29.02.2012**
(21) Anmeldenummer: 06723619.0
(22) Anmeldetag: 22.03.2006
(51) Int. Cl.: A61M 1/00

(54) **VAKUUMTHERAPIEVORRICHTUNG**
VACUUM THERAPY DEVICE
DISPOSITIF DE THERAPIE SOUS VIDE

(30) Priorität: 24.03.2005 DE 102005014420
(43) Veröffentlichungstag der Anmeldung: 12.12.2007
(73) Patentinhaber: VCS Medical Technology GmbH, 22525 Hamburg (DE)
(72) Erfinder: MEYER, Johannes, 20459 Hamburg (DE); BAUMANN, Dirk, 20459 Hamburg (DE)
(74) Vertreter: Kloiber, Thomas
(86) Internationale Anmeldenummer: PCT/EP2006/002623
(87) Internationale Veröffentlichungsnummer: WO 2006/100053

(56) Entgegenhaltungen:
- WO-A-96/05873
- WO-A-2004/037334
- US-A- 6 142 982
- US-A1- 2003 040 687
- US-B1- 6 398 767

## Beschreibung

Die vorliegende Erfindung betrifft ein Verfahren zum Betreiben eines therapeutischen Gerätes zur Förderung der Heilung einer Wunde, umfassend,
a. eine im Wesentlichen luftdichte Abdeckung um die Wunde herum, wobei zwischen Wunde und Abdeckung vorzugsweise ein für Flüssigkeiten durchlässiges Polster angeordnet ist,
b. eine die Abdeckung mit einer Saugpumpe verbindende Drainageleitung, so dass an der Wunde gesaugt werden kann, um die Flüssigkeiten von dort abzuziehen, wobei ein distales Ende der Drainageleitung mit der Saugpumpe indirekt über ein Reservoir zum Sammeln der von der Wunde abgesaugten Flüssigkeit verbunden ist,
c. Drucksensormittel zur direkten oder indirekten Bestimmung eines im Bereich zwischen der Wunde und der Abdeckung herrschenden Wunddrucks und
d. eine Regelungseinheit zur Regelung des Wunddrucks.

Wundheilvorrichtungen, die im Rahmen derartiger Verfahren verwendet werden, dienen zur Behandlung sekundär verheilender chronischer oder akuter Wunden, insbesondere beim Menschen. Mit Hilfe des Unterdrucks um den die Wunde beinhaltenden Hautbereich soll Flüssigkeit, also insbesondere Wundsekret, von der Wunde wegtransportiert werden, um somit die Heilungsdauer zu verkürzen. In vielen Fällen wird durch den Einsatz einer derartigen Wundheilvorrichtung die Wundheilung überhaupt erst möglich gemacht.

Eine gattungsgemäße Wundheilvorrichtung ist z. B. in der US 6,142,982 A beschrieben. Bei dem dort beschriebenen Gerät zur Förderung der Wundheilung ist insbesondere vorgesehen, dass das Gerät eine zusätzliche Leitung beinhaltet, die das poröse Polster mit einem Drucknachweisgerät verbindet. Mithilfe der zusätzlichen Leitung kann der Druck an dem Ort der Wunde überwacht werden. Zu diesem Zweck soll bei dem vorbekannten Therapiegerät der Drucksensor in dem Gehäuse der Wundheilvorrichtung angeordnet sein. Mit Hilfe einer ebenfalls im Gehäuse angeordneten Regelung soll der mittels der zusätzlichen Leitung gemessene Wunddruck auf einen gewünschten Wert eingeregelt werden.

Nachteilig an diesem bekannten Wundheilgerät ist, dass es vergleichsweise aufwendig ist, neben der Drainageleitung eine zusätzliche Schlauchleitung vorzusehen. Ein solcher Schlauch erhöht allgemein die Komplexität der Apparatur. Zwar ist vorgeschlagen worden, einen Multilumenschlauch zu verwenden, dessen Hauptröhre als Drainageschlauch verwendet wird und von dem eine Nebenröhre als Sondenschlauch für die Druckmessung dient. Hierdurch wird jedoch beispielsweise der Außendurchmesser der Schlauchverbindung zwischen Gehäuse und Wundauflage in unerwünschter Weise vergrößert. Auch ist nicht auszuschließen, dass die zusätzliche Leitung verstopft oder z. B. durch Knicken oder auf andere Weise vom am Wundort herrschenden Druck durch Drosselwirkung abgetrennt ist. In einem solchen Falle würde die Druckmessung bei dem bekannten Therapiegerät einen falschen Wert liefern, ohne dass dies auf eine Störung im Drainageschlauch oder am Wundort zurückzuführen wäre. Dementsprechend würde die Regelung des Wunddrucks versagen.

Auch in der WO 2004/037 334 ist eine gattungsgemäße Wundheilvorrichtung sowie ein gattungsgemäßes Verfahren beschrieben. Das dort beschriebene Gerät dient zur Belüftung, zum Spülen und Reinigen von Wunden. Eine Spülflüssigkeit wird von einem Reservoir, welches mit einer formanpassungsfähigen Wundauflage verbunden ist, sowie Flüssigkeit von der Wundauflage mittels einer peristaltischen Pumpe durch Schläuche in einem Kreislauf gepumpt. Dabei ist die Wundauflage geeignet, eine relativ flüssigkeitsdichte Abdichtung der Wunde herzustellen. Die Pumpe wird von einem Regler angesteuert, wobei der Regler als Eingangsgröße Druckmesswerte eines Drucksensors, der sich unter der Wundabdeckung befindet, erhält.

Nachteilig an diesem bekannten Wundheilgerät ist, wie auch schon in der US 6,142,982 A ausgeführt, dass es vergleichsweise aufwendig ist, einen zusätzlichen Drucksensor unter der Wundabdeckung anzuordnen.

Der vorliegenden Erfindung liegt daher die Aufgabe zugrunde, ein gattungsgemäßes Verfahren zum Betreiben eines therapeutischen Gerätes zur Förderung der Heilung einer Wunde anzugeben, das mit einfachen Maßnahmen eine zuverlässige und sichere Wundversorgung ermöglicht.

Die Erfindung löst diese Aufgabe durch ein Verfahren, bei dem zur Bestimmung des Wunddrucks bei gegebener Saugleistung der Saugpumpe zunächst mit einem an dem distalen Ende des Drainageschlauches angeordneten Drucksensor ein Distaldruck gemessen wird und diesem anschließend mit einer in der Regelungseinheit abgespeicherten Kalibrierungstabelle einem aus dem Distaldruck und der Saugleistung gebildeten Wertepaar ein Druckwert berechnet wird, dessen Höhe im Wesentlichen dem Wunddruck entspricht.

Mit Vorteil benötigt das erfindungsgemäße Verfahren keine Druckmessung am Ort der Wunde, so dass ein entsprechender Drucksensor und ggf. zusätzlicher Schlauch entfallen kann. Dabei ist dennoch eine kontinuierliche Bestimmung des Drucks möglich. Die Druckbestimmung wird dadurch ermöglicht, dass ein durch einen Strömungswiderstand im Drainageschlauch erzeugter Druckabfall auf dem Wege der Kalibration bei der Messung berücksichtigt wird. Auf diese Weise kann der Wunddruck bestimmt werden, ohne dass am Ort der Wunde, also etwa am Polster, ein Drucksensor angeordnet ist. Desweiteren muss auch kein Sondenschlauch o. ä. zur Wunde geführt werden, um die Messung des Wunddrucks zu ermöglichen. Die Auswertung des vom am distalen Ende des Drainageschlauchs angeordneten Drucksensor gemessenen Distaldrucks kann problemlos in der Regelungseinheit erfolgen. Dort ist die Kalibrationstabelle abgelegt, mit Hilfe derer der Distaldruck in einen Wunddruck umgerechnet wird. Durch die Kalibrationstabelle wird dabei jedem Wertepaar aus Saugleistung der Saugpumpe und innerhalb der luftdichten Abdeckung gemessenem Wunddruck einen Distaldruck zugeordnet. Die einfache Zuordnung eines Wunddrucks lediglich zum Distaldruck, ohne dabei die Pumpleistung zu berücksichtigen, wäre demgegenüber bei laufender Pumpe aufgrund der Saugleistungsabhängigkeit der dynamischen Druckgradienten nicht ausreichend. Innerhalb der Regelungseinheit sollten geeignete Interpolationsalgorithmen hinterlegt sein, zur Berechnung der Zwischenwerte der Tabelle.

In vorteilhafter Ausgestaltung des Verfahrens ist vorgesehen, dass die Bestimmung der Kalibrierungstabelle die folgenden Schritte umfasst:
a. die Saugpumpe wird mit verschiedenen vorgegebenen konstanten Saugleistungen betrieben;
b. für jede Saugleistung wird gleichzeitig der Wunddruck mittels eines innerhalb der luftdichten Abdeckung angeordneten Wunddrucksensors (30) und der Distaldruck (p1) mittels eines am distalen Ende des Drainageschlauches (20) angeordneten Distaldrucksensors gemessen;
c. der gemessene Wunddruck (p3) und Distaldruck (p1) sowie die zugehörige Saugleistung werden jeweils als Wertetripel in die Kalibrierungstabelle eingetragen.

Dieses Verfahren zur Ermittlung der Kalibrierungstabelle ist besonders gut geeignet, um den benötigten Wertebereich systematisch zu erfassen.

Die Erfindung wird auch verbessert mit Hilfe eines Verfahrens zum Ermitteln von Fehlern im Drainageschlauch, welches folgende Schritte umfasst:
d. gleichzeitiges direktes Messen des Wunddrucks bei laufender Saugpumpe innerhalb der luftdichten Abdeckung sowie des Distaldrucks am distalen Ende des Drainageschlauches;
e. Berechnen einer Differenz zwischen dem Wunddruck und dem Distaldruck;
f. Vergleichen der Differenz mit einer Referenzdifferenz;
g. Feststellen eines Fehlers im Drainageschlauch bei Abweichen von der Referenzdifferenz um einen vorbestimmten Schwellwert.

Durch dieses Verfahren können mit Vorteil z. B. Verstopfungen im Drainageschlauch erfasst werden. Außerdem ist es mit dem erfindungsgemäßen Verfahren möglich, festzustellen, dass der Drainageschlauch abgeknickt ist und dadurch in unerwünschter Weise als Drossel für den Druck wirkt. Insbesondere ist es vorteilhaft bei diesem Verfahren, dass der Wunddruck letztlich gleichzeitig durch zwei verschiedene Verfahren gemessen wird. Dabei ist die direkte Messung des Wunddrucks innerhalb der luftdichten Abdeckung unbeeinflusst von durch dynamische Effekte bedingten Druckgradienten im Drainageschlauch. Demgegenüber ist auf der anderen Seite der Distaldruck am distalen Ende des Drainageschlauches von den dynamischen Effekten im Drainageschlauch beeinflusst. Ein Messen beider Druckgrößen zum selben Zeitpunkt ermöglicht es daher gemäß der Erfindung, Änderungen des dynamischen Druckabfalls von anderen Effekten zu isolieren. Sofern sich nämlich der Distaldruck ändert, ohne dass sich der Wunddruck oder die Saugleistung der Saugpumpe ändern, ist auf einen Fehler im Drainageschlauch, wie beispielsweise eine Verstopfung mit Wundsekret o. ä. oder ein Abknicken des Schlauches zu schließen. Zur optimalen Versorgung des Patienten ist diese Verbesserung in der Messtechnik des Vakuumtherapiegerätes ein enormer Vorteil.

Entsprechend einer besonders vorteilhaften Ausführungsform des obigen erfindungsgemäßen Verfahrens ist vorgesehen, dass der Wunddruck nur qualitativ ausgewertet wird. Hierdurch kann zur Messung des Wunddrucks ein besonders kostengünstiger Sensor - etwa ein Dehnungsmessstreifen - verwendet werden, der lediglich als Druckschalter verwendet wird, um beispielsweise das Überschreiten eines für die Therapie mindestens erforderlichen Unterdrucks an der Wunde zu detektieren. Insbesondere, für den Fall, dass die Wundauflageeinheit gemeinsam mit dem

Wunddrucksensor als Einmalartikel konzipiert ist, ergibt sich hierdurch ein erhebliches Kosteneinsparpotential.

Die Erfindung wird in einer bevorzugten Ausführungsform unter Bezugnahme auf eine Zeichnung beispielhaft beschrieben, wobei weitere vorteilhafte Einzelheiten den Figuren der Zeichnung zu entnehmen sind.

Funktionsmäßig gleiche Teile sind dabei mit denselben Bezugszeichen versehen.

Die Figuren der Zeichnung zeigen im Einzelnen:
- Fig. 1: schematische Darstellung der erfindungsgemäßen Wundheilvorrichtung sowie deren Funktionsweise
- Fig. 2: Detaildarstellung der erfindungsgemäßen Wundheilvorrichtung mit dem Absaugschlauch

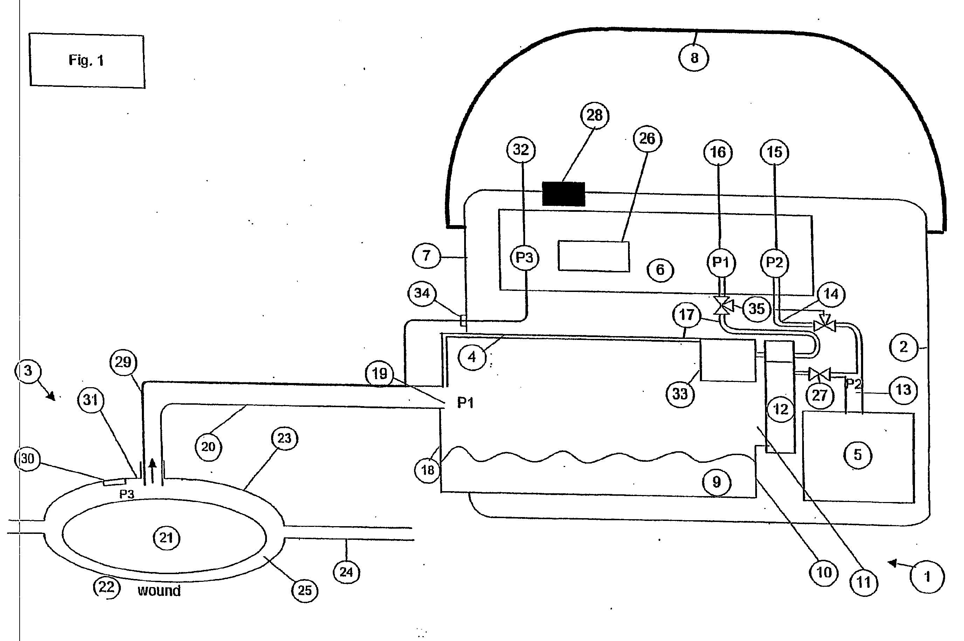

Die Figur 1 zeigt schematisch ein Ausführungsbeispiel einer erfindungsgemäßen Wundheilvorrichtung 1. Die Wundheilvorrichtung 1 besteht aus einer Basiseinheit 2 und einer Wundauflageeinheit 3. In der Basiseinheit 2 befindet sich ein wechselbarer Auffangbehälter 4, eine Saugpumpe 5 sowie ein Regel- und Steuergerät 6. Die Basiseinheit 2 ist von einem Gehäuse 7 umgeben. An dem Gehäuse 7 ist ein Tragegurt 8 angebracht.

In dem Auffangbehälter 4 befindet sich ein Gemisch 9 aus Wundsekret und Geliermittel. Der Auffangbehälter 4 weist an einer dem Gehäuse 7 zugewandten Innenwand 10 eine Absaugöffnung 11 auf. Die Absaugöffnung 11 ist über den Filter 12 über einen Absaugschlauch 13 mit der Saugpumpe 5 verbunden. Von dem Absaugschlauch 13 ist ein pumpenseitiger Sondenschlauch 14 abgezweigt. Der pumpenseitige Sondenschlauch 14 führt den im Absaugschlauch 13 herrschenden Druck p2 zu einem saugpumpenseitigen Drucksensor 15, welcher sich in dem Regel- und Steuergerät 6 befindet. In dem Regel- und Steuergerät 6 befindet sich ferner der Distaldrucksensor 16, welcher über den Sondenschlauch 17 sowie den Distalsondenschlauch 36 mit dem am distalen Ende des Absaugschlauches 13 herrschenden Druck p1 kommuniziert. Sowohl in dem Sondenschlauch 17 als auch in dem Sondenschlauch 14 ist jeweils ein 3/2-Wegeventil 35 eingebaut. Der Distalsondenschlauch 36 ist entlang des wechselbaren Auffangbehälters 4 innerhalb des Gehäuses 7 zu einer in einer Außenwand 18 des Auffangbehälters 4 befindlichen Ansaugöffnung 19 im Gehäuse 7 der Basiseinheit 2 geführt. Außerdem kann der Distalsondenschlauch 36 optional durch eine Blackbox 33 geführt sein.

Die Wundauflageeinheit 3 besteht aus dem Wundsekretabsaugschlauch 20, einem Schwamm 21 sowie einer Folie 23, durch welche der Wundsekretabsaugschlauch 20 und der Schwamm 21 so auf die die Wunde 22 umgebende Haut 24 aufgeklebt sind, dass eine luftdichte Verbindung entsteht. Der Schwamm 21 ist in der Lage, Flüssigkeiten aufzunehmen und zu speichern. Der Schwamm 21 wird durch die gespannte Folie 23 mit einem gewissen Anpressdruck in die Wunde 22 eingebracht.

In der Wundauflage 3 ist ferner zwischen der Folie 23 und dem Schwamm 21 in der Nähe des Schlauchanschlusses 31 ein Dehnungsmeßstreifen 30 befestigt. Der Dehnungsmeßstreifen 30 dient zur Messung des Wunddrucks an der Wunde 22. Der Dehnungsmeßstreifen 30 ist mit einem elektrischen Kabel 29 verbunden. Das elektrische Kabel 29 ist mit dem äußeren Rand des Absaugschlauches 20 verbunden und auf diese Weise mittels einer Steckverbindung 34 im Gehäuse 7 der Basiseinheit 2 mit dem Auswertegerät 32 für den Wunddruck P3 verbunden. Das Auswertegerät 32 ist Bestandteil des Regel- und Steuergerätes 6.

Der Auffangbehälter 4, der Wundsekretabsaugschlauch 20, der Schwamm 21 und die Folie 23 sind jeweils steril und auswechselbar zum Einmalgebrauch. In der Wunde 22 befindet sich Wundsekret 25.

Zur Verwendung der erfindungsgemäßen Wundheilvorrichtung 1 wird der Schwamm 21 in die Wunde 22 eingebracht. Mit dem Schwamm 21 wird ein Ende des Wundsekretabsaugschlauchs 20 verbunden und anschließend werden sowohl der Wundsekretabsaugschlauch 20 als auch der Schwamm 21 mit Hilfe der Folie 23 auf die die Wunde 22 umgebende Haut 24 derart aufgeklebt, dass dieses System luftdicht abgeschlossen ist.

Das andere Ende des Wundsekretabsaugschlauchs 20 wird mit der in der Außenwand 18 des Auffangbehälters 4 befindlichen Ansaugöffnung 19 ebenfalls luftdicht verbunden. Durch die Eigenschaft des Schwammes 21, Flüssigkeiten aufnehmen und speichern zu können, wird Wundsekret 25 aus der Wunde 22 in den Schwamm 21 abgeführt. Die die Speicherkapazität des Schwammes 21 übersteigende Wundsekretmenge wird nun über den Wundsekretabsaugschlauch 20 in den Auffangbehälter 4 abgesaugt. Der für die Absaugung erforderliche Unterdruck wird dabei von der Absaugpumpe 5 erzeugt.

Dazu kommuniziert der Absaugschlauch 13 der Saugpumpe 5 mit dem Auffangbehälter 4. Der zwischen dem Auffangbehälter 4 und dem Absaugschlauch 13 angeordnete Filter 12 verhindert dabei, dass Wundsekret 25 aus dem Auffangbehälter 4 in den Absaugschlauch 13, die Absaugpumpe 5, in den saugpumpenseitigen Sondenschlauch 14 oder gar in den saugpumpenseitigen Drucksensor 15 eintritt.

Das auf die beschriebene Weise in dem Auffangbehälter 4 gesammelte überschüssige Wundsekret 25 wird in dem Auffangbehälter 4 durch ein Geliermittel verdickt. Hierdurch wird ein Befeuchten des Filters 12 auch bei Lagewechsel der Basiseinheit 2 wirksam verhindert. Dadurch wird z.B. verhindert, dass der Filter 12 befeuchtet wird und ein Übertritt von Keimen in das Steuergerät 6 erfolgen könnte. Außerdem ist eine hygienischere Entsorgung der potentiell verunreinigten Wundflüssigkeit im festen Aggregatzustand möglich.

Der von der Saugpumpe 5 erzeugte Unterdruck wird von dem saugpumpenseitigen Drucksensor 15 einerseits sowie von dem wundseitigen Dehungsmeßstreifen 30 andererseits und außerdem über den Distaldruckaufnehmer 16, welcher den Distaldruck p1 mißt, kontinuierlich aufgenommen. In dem Mikroprozessor des Regel- und Steuergerätes 6 wird anschließend eine Auswertung des durch den wundseitigen Dehnungsmeßstreifen 30 aufgenommenen Drucks p3, des durch den saugpumpenseitigen Drucksensor 15 aufgenommenen Drucks p2 sowie des am distalen Ende des Absaugschlauchs 20 aufgenommenen Drucks p1 als Eingangsgrößen für die Regelung ausgewertet.

Zur Aufrechterhaltung eines feuchten Wundmilieus wird das Druckniveau in der Wundauflageeinheit 3 nach den individuellen Bedürfnissen des Patienten sowohl bezüglich der Eigenschaften der Wunde 22 als auch nach Kriterien des Verträglichkeitsempfindens (Schmerzen des Patienten) eingestellt. Die Einstellung erfolgt dabei über eine Softwaremenüführung auf einer Anzeigeeinheit 26 auf dem mikroprozessorgesteuerten Regel-und Steuergerät 6. Die Softwaremenüführung erlaubt es dabei, verschiedene Sprachen für die Benutzerführung auszuwählen. Außerdem kann über die Softwaremenüführung das gewünschte Regelprogramm für den Unterdruck an der Wundauflageeinheit 3 ausgewählt werden. Dabei verarbeitet der Mikroprozessor des Regel- und Steuergerätes 6 die Signale des wundseitigen Dehnungsmeßstreifens 30 - also das Drucksignal p3 - , des saugpumpenseitigen Drucksensors 15 - also das Drucksignal p2 - sowie des Distaldrucksensors 16 - also das Drucksignal p1 - gemäß dem ausgewählten Regelalgorithmus zu Ausgangsgrößen. Die Ausgangsgrößen werden von dem Regel- und Steuergerät 6 an das Regelventil 27 und eventuell auch direkt an die Saugpumpe 5 geleitet.

Mit Hilfe des Regelventils 27, welches sich in dem Absaugschlauch 13 zwischen der Saugpumpe 5 und dem Filter 12 befindet, lassen sich verschiedene Druckregelprogramme physisch realisieren, da dass Regelventil 27 schnell geöffnet und geschlossen werden kann, gemäß den Steuersignalen des Regel- und Steuergerätes 6.

Über die Anzeigeeinheit 26 und die Softwaremenüführung kann der Benutzer auch ein festes Druckregelprogramm ablaufen lassen. Das Regel- und Steuergerät 6 beeinflußt dabei unter Auswertung der an den Drucksensoren 15, 16, 30/32 ankommenden Drucksignale p1, p2, p3 das Ventil 27 und / oder die Saugpumpe 5 als Stellgrößen für die Druckbereiche und Steuerungszeiten. Im Sinne einer Regelung sind nur geringe Abweichungen des Ist-Wertes vom Soll-Wert zulässig.

Mit dem Tragegurt 8 kann die Basiseinheit 2 von dem Patienten, auf dessen Wunde 22 die Wundauflageeinheit 3 angebracht ist, getragen werden. Der Patient ist dadurch mobil auch während der Benutzung der erfindungsgemäßen Wundheilvorrichtung 1.

Zur Wartung und für Patientendokumentationszwecke ist das Regel- und Steuergerät 6 mit einem Computerinterface 28 ausgestattet. Hierdurch können beispielsweise über einen externen Rechner neue Regelalgorithmen eingespielt werden oder Patientendaten an eine zentrale Verwaltung weitergeleitet werden.

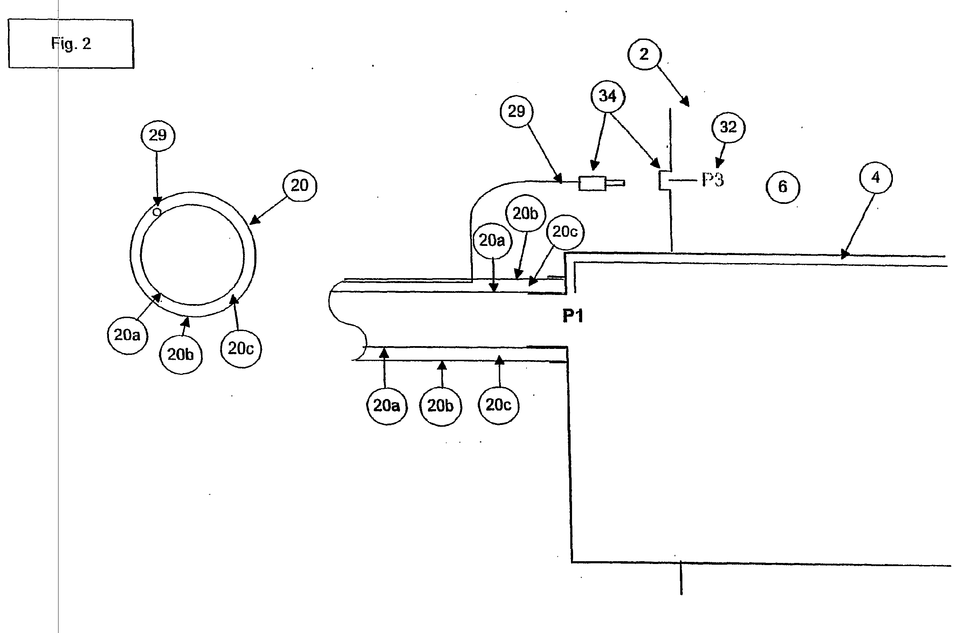

In der Figur 2 ist eine bevorzugte Möglichkeit dargestellt, um das elektrische Kabel 29 zur Basiseinheit 2 zu führen. Wie in der Figur zu erkennen, weist der im Querschnitt dargestellte Absaugschlauch 20 eine innere 20a und eine äußere 20b Schlauchwand auf. In dem Hohlraum 20c zwischen der inneren 20a und der äußeren 20b Schlauchwand ist das elektrische Kabel 29 geführt, welches den Dehnungsmeßstreifen 30 mit der Regel- und Steuereinheit 6 in der Basiseinheit 2 verbindet. In der Nähe der Basiseinheit 2 ist das elektrische Kabel 29 aus dem Hohlraum 20c herausgeführt und über die Steckverbindung 34 mit dem Regel- und Steuergerät in der Basiseinheit 2 verbunden. Auf diese Weise wird das Signal des Dehnungsmeßstreifens 30 an das Auswertegerät 32 für den Wunddruck p3 übertragen. '

Mit dem Gerät lassen sich nun die folgenden verschiedenen Verfahren der Messung des Wunddrucks durchführen:

Zunächst kann der Druck anhand des Dehnungsmeßstreifens 30 direkt am Ort der Wunde innerhalb der luftdichten Abdeckung der Wunde herum erfolgen. Die Auswertung des Meßwertes p3 für den Wunddruck des Dehungsmeßstreifens 30 erfolgt dabei anhand des Auswertegerätes 32 innerhalb des Regelungs- und Steuergerätes 6.

Ferner kann der Wunddruck durch Messung des Distaldrucks p1 mit dem Distaldrucksensor 16, welcher den Druck am distalen Ende des Absaugschlauches 20 erfaßt, ermittelt werden. Dazu wird der dynamisch durch die Absaugströmung bedingte Druckgradient durch Kalibrierung mit Hilfe des Regelungs- und Steuergerätes 6 berücksichtigt. Die Kalibrierung erfolgt dabei durch Auswertung des von dem Dehnungsmeßstreifen 30 gemessenen Wunddrucks p3 in Verbindung mit den zugehörigen Werten für p1. Dieses Wertepaar muß für verschiedene Pumpleistungen ermittelt werden und in der Kalibrierungskurve abgelegt werden. Nachdem die Kalibrierungskurve bestimmt worden ist, ist der Dehnungsmeßstreifen 30 am Ort der Wunde für die Messung nicht mehr erforderlich. Die Wundauflage könnte daher im Normalfall ohne Meßtechnik geliefert werden. Lediglich für Kalibrierungszwecke könnte eine Kalibrierungswundauflage verwendet werden, welche einen Dehnungsmeßstreifen oder anderen Drucksensor aufweist. Auf diese Weise können mit Vorteil die Herstellungskosten der üblicherweise als Einmalartikel zu verwendenden Wundauflagen niedrig gehalten werden.

Schließlich kann der Wunddruck auch durch Messung des Distaldrucks p1 bestimmt werden, ohne daß eine Berücksichtigung dynamischer Effekte berücksichtigt werden muß. Dazu wird vor jeder Messung die Saugpumpe 5 abgeschaltet und eine Relaxationszeit abgewartet. Innerhalb der Relaxationszeit bildet sich ein stationärer Zustand aus, bei dem der Druck p3 am Ort der Wunde derselbe ist wie der Druck p1 am distalen Ende des Drainageschlauches 20. Eine einfache Messung des Drucks p1 durch den Drucksensor 16 über den Sondenschlauch 36 liefert in diesem Falle bereits den gesuchten Wunddruck. Um eine Regelung durchführen zu können, sollte diese Messung in regelmäßigen Abständen durchgeführt werden.

Darüber hinaus können die beschriebenen Verfahren zur Messung des Wunddrucks auch gleichzeitig eingesetzt werden. Hierdurch kann zum einen der Vertrauensbereich der Messung verbessert werden. Außerdem lassen sich auf diese Weise auch Fehlerdiagnoseverfahren durchführen, um beispielsweise eine Verstopfung des Drainageschlauches festzustellen. Eine Verstopfung liegt dann vor, wenn bei gleichbleibendem Meßwert p3 eine Änderung des Drucks p1 festgestellt wird, wobei p1 entweder mit der dynamischen oder mit der statischen Meßmethode gemessen werden kann, wie oben beschrieben.

Um eine Verstopfung des Filters 12 festzustellen, kann der im Absaugschlauch 13 herrschende Druck p2 an der Saugseite der Saugpumpe 5 mit Hilfe des saugpumpenseitigen Drucksensors 15 und gleichzeitig mit dem Distaldrucksensor 16 über den Sondenschlauch 17 sowie den Distalsondenschlauch 36 den am distalen Ende des Absaugschlauches 13 herrschenden Druck p1 gemessen werden. Die im Regel- und Steuergerät 6 ausgewertete Differenz von p1 und p2 korreliert dann mit dem Verstopfungsgrad des Filters. Bei Erreichen eines bestimmten Verstopfungsgrades kann der Benutzer zum Auswechseln des Filters aufgefordert werden.

Ferner kann die Messung des Drucks p2 auch zur Messung der Saugleistung der Saugpumpe 5 verwendet werden.

Somit ist eine Vorrichtung zur Förderung der Wundheilung vorgeschlagen, bei welcher eine deutliche Verbesserung der Meßtechnik realisiert wurde und mit welcher infolgedessen eine gegenüber herkömmlichen Vorrichtungen stark verbesserte und verläßlichere Regelung des Wunddrucks möglich ist.

### BEZUGSZEICHENLISTE

- 1: Wundheilvorrichtung
- 2: Basiseinheit
- 3: Wundauflageeinheit
- 4: Auffangbehälter
- 5: Saugpumpe
- 6: Regel- und Steuergerät
- 7: Gehäuse
- 8: Tragegurt
- 9: Gemisch
- 10: Innenwand
- 11: Absaugöffnung
- 12: Filter
- 13: Absaugschlauch
- 14: saugpumpenseitiger Sondenschlauch
- 15: saugpumpenseitiger Drucksensor
- 16: wundseitiger Drucksensor
- 17: wundseitiger Sondenschlauch
- 18: Außenwand
- 19: Ansaugöffnung
- 20: Wundsekretabsaugschlauch
- 20a: innere Schlauchwand
- 20b: äußere Schlauchwand
- 20c: Hohlraum
- 21: Schwamm
- 22: Wunde
- 23: Folie
- 24: Haut
- 25: Wundsekret
- 26: Anzeigeeinheit
- 27: Regelventil
- 28: Computerinterface
- 29: elektrisches Kabel
- 30: Dehnungsmeßstreifen
- 31: Schlauchanschluß
- 32: P3 Auswertegerät
- 33: Blackbox
- 34: Steckverbindung
- 35: 3/2 Wegeventil
- 36: distaler Sondenschlauch

## Patentansprüche

1. Verfahren zum Betreiben eines therapeutischen Gerätes (1) zur Förderung der Heilung einer Wunde (22), umfassend,
a. eine im wesentlichen luftdichte Abdeckung (23) um die Wunde (22) herum, wobei zwischen Wunde (22) und Abdeckung (23) vorzugsweise ein für Flüssigkeiten durchlässiges Polster (21) angeordnet ist,
b. eine die Abdeckung (21) mit einer Saugpumpe (5) verbindende Drainageleitung (20), so dass an der Wunde (22) gesaugt werden kann, um die Flüssigkeiten von dort abzuziehen, wobei ein distales Ende der Drainageleitung (20) mit der Saugpumpe (5) indirekt über ein Reservoir (4) zum Sammeln der von der Wunde (22) abgesaugten Flüssigkeit verbunden ist,
c. Drucksensormittel (30, 16, 32) zur direkten oder indirekten Bestimmung eines im Bereich zwischen der Wunde (22) und der Abdeckung (23) herrschenden Wunddrucks (p3) und
d. eine Regelungseinheit (6) zur Regelung des Wunddrucks,
**dadurch gekennzeichnet, dass** zur Bestimmung des Wunddrucks (p3) bei gegebener Saugleistung der Saugpumpe (5) zunächst mit einem an dem distalen Ende des Drainageschlauches (20) angeordneten Drucksensor (16) ein Distaldruck (p1) gemessen wird und diesem anschließend mit einer in der Regelungseinheit (6) abgespeicherten Kalibrierungstabelle einem aus dem Distaldruck (p1) und der Saugleistung gebildeten Wertepaar ein Druckwert berechnet wird, dessen Höhe im wesentlichen dem Wunddruck (p3) entspricht.

2. Verfahren nach Anspruch 1,**dadurch gekennzeichnet, dass** die Bestimmung einer Kalibrierungstabelle die folgenden Schritte umfasst:
a. die Saugpumpe (5) wird mit verschiedenen vorgegebenen konstanten Saugleistungen betrieben;
b. für jede Saugleistung wird gleichzeitig der Wunddruck (p3) mittels eines innerhalb der luftdichten Abdeckung (23) angeordneten Wunddrucksensors (30) und der Distaldruck (p1) mittels eines am distalen Ende des Drainageschlauches (20) angeordneten Distaldrucksensors gemessen;
c. der gemessene Wunddruck (p3) und Distaldruck (p1) sowie die zugehörige Saugleistung werden jeweils als Wertetripel in die Kalibrierungstabelle eingetragen.

3. Verfahren nach einem der Ansprüche 1 oder 2,
**dadurch gekennzeichnet, dass** es zum Ermitteln von Fehlern im Drainageschlauch (20) folgende Schritte umfasst:
a. gleichzeitiges direktes Messen des Wunddrucks (p3) bei laufender Saugpumpe (5) innerhalb der luftdichten Abdeckung (23) sowie des Distaldrucks (p1) am distalen Ende des Drainageschlauches (20);
b. Berechnen einer Differenz zwischen dem Wunddruck (p3) und dem Distaldruck (p1);
c. Vergleichen der Differenz mit einer Referenzdifferenz;
d. Feststellen eines Fehlers im Drainageschlauch (20) bei Abweichen von der Referenzdifferenz um einen vorbestimmten Schwellwert.

4. Verfahren nach Anspruch 3, **dadurch gekennzeichnet, dass** der Wunddruck (p3) nur qualitativ ausgewertet wird.

## Claims

1. Method for operating a therapeutic device (1) for promoting the healing of a wound (22), comprising,
a. a substantially airtight cover (23) around the wound (22), a liquid-permeable pad (21) preferably being arranged between the wound (22) and the cover (23),
b. a drainage tube (20) connecting the cover (21) to a suction pump (5) so that suction can be applied to the wound (22) in order to draw fluids therefrom, wherein a distal end of the drainage tube (20) is connected indirectly to the suction pump (5) via a reservoir (4) for collecting the fluid suctioned from the wound (22),
c. pressure sensor means (30, 16, 32) for directly or indirectly determining a prevailing wound pressure (p3) in the region between the wound (22) and the cover (23), and
d. a control unit (6) for controlling the wound pressure,
**characterised in that**, for determining the wound pressure (p3) at a given suction power of the suction pump (5), firstly a distal pressure (p1) is measured using a pressure sensor (16) arranged on the distal end of the drainage tube (20) and subsequently a pressure value substantially corresponding to the wound pressure (p3) is calculated from a pair of values consisting of the distal pressure (p1) and the suction power using a calibration table stored in a control unit (6).

2. Method according to claim 1, **characterised in that** determining a calibration table comprises the following steps:
a. operating the suction pump (5) at various predetermined constant suction powers;
b. for each suction power, simultaneously measuring the wound pressure (p3) using a wound pressure sensor (30) arranged inside the airtight cover (23) and the distal pressure (p1) using a distal pressure sensor arranged on the distal end of the drainage tube (20);
c. entering the measured wound pressure (p3) and distal pressure (p1) and the relevant suction power into the calibration table as a triple.

3. Method according to either claim 1 or claim 2, **characterised in that** it comprises the following steps for detecting errors in the drainage tube (20):
a. simultaneously directly measuring the wound pressure (p3) while the suction pump (5) is running inside the airtight cover (23) and the distal pressure (p1) on the distal end of the drainage tube (20);
b. calculating the difference between the wound pressure (p3) and the distal pressure (p1);
c. comparing the difference with a reference difference;
d. detecting an error in the drainage tube (20) in the event of deviation from the reference difference by a predetermined threshold value.

4. Method according to claim 3, **characterised in that** the wound pressure (p3) is only qualitatively evaluated.

## Revendications

1. Procédé pour faire fonctionner un appareil thérapeutique (1) servant à activer la guérison d'une plaie (22), comportant
a. un revêtement (23) essentiellement hermétique se situant autour de la plaie (22), un rembourrage (21) perméable aux liquides étant de préférence disposé entre la plaie (22) et le revêtement (23),
b. un drain (20) reliant le revêtement (21) à une pompe d'aspiration (5) de telle sorte qu'il est possible d'aspirer au niveau de la plaie (22) afin d'en éliminer les liquides, une extrémité distale du drain (20) étant reliée à la pompe d'aspiration (5) indirectement par l'intermédiaire d'un réservoir (4) pour collecter le liquide aspiré de la plaie (22),
c. des moyens capteurs de pression (30, 16, 32) pour déterminer de manière directe ou indirecte une pression de la plaie (p3) régnant dans la zone entre la plaie (22) et le revêtement (23) et
d. une unité de réglage (6) pour régler la pression de la plaie,
**caractérisé en ce que** pour déterminer la pression d'une plaie (p3) avec une capacité d'aspiration donnée d'une pompe d'aspiration (5), on mesure dans un premier temps avec un capteur de pression (16) disposé à l'extrémité distale du tube flexible de drainage (20), une pression distale (p1) puis on calcule à l'aide d'une nomenclature de calibrage mémorisée dans l'unité de réglage (6), à partir d'une paire de valeurs formée à partir de la pression distale (p1) et de la capacité d'aspiration, une valeur de pression, qui correspond essentiellement à la pression de la plaie (p3).

2. Procédé selon la revendication 1, **caractérisé en ce que** la détermination d'une nomenclature de calibrage comporte les étapes suivantes :
a. la pompe d'aspiration (5) fonctionne avec diverses capacités d'aspiration constantes qui sont prédéfinies ;
b. pour chaque capacité d'aspiration, on mesure en même temps la pression de la plaie (p3) au moyen d'un capteur de pression de la plaie (30) disposé à l'intérieur du revêtement (23) hermétique, et la pression distale (p1) au moyen d'un capteur de pression distale disposé au niveau de l'extrémité distale du tuyau flexible de drainage (20) ;
c. la pression de la plaie (p3) mesurée et la pression distale (p1) ainsi que la capacité d'aspiration associée sont enregistrées respectivement en tant que valeur triple dans la nomenclature de calibrage.

3. Procédé selon l'une quelconque des revendications 1 ou 2,
**caractérisé en ce qu'**il comprend pour détecter des erreurs dans le tuyau flexible de drainage (20) les étapes suivantes consistant à :
a. mesurer directement en même temps la pression de la plaie (p3) lors du fonctionnement de la pompe d'aspiration (5) à l'intérieur du revêtement (23) hermétique, ainsi que la pression distale (p1) au niveau de l'extrémité distale du tuyau flexible de drainage (20) ;
b. calculer une différence entre la pression de la plaie (p3) et la pression distale (p1) ;
c. comparer ladite différence à une différence de référence ;
d. déterminer une erreur dans le tuyau flexible de drainage (20) en cas d'écart avec la différence de référence d'une valeur seuil prédéfinie.

4. Procédé selon la revendication 3, **caractérisé en ce que** la pression de la plaie (p3) est évaluée seulement d'un point de vue qualitatif.
